Europäisches Patentamt

European Patent Office

Office européen des brevets

(19)

(11) Publication number : **0 299 082 B1**

# (12) EUROPEAN PATENT SPECIFICATION

(45) Date of publication of patent specification :
06.05.92 Bulletin 92/19

(51) Int. Cl.⁵ : **A61K 31/71,** // A61K39/12,
C07H21/04

(21) Application number : 88901107.8

(22) Date of filing : 25.01.88

(86) International application number :
PCT/JP88/00054

(87) International publication number :
WO 88/05300 28.07.88 Gazette 88/17

(54) ANTIVIRAL AGENT.

(30) Priority : 27.01.87 JP 16408/87
25.12.87 JP 329272/87

(43) Date of publication of application :
18.01.89 Bulletin 89/03

(45) Publication of the grant of the patent :
06.05.92 Bulletin 92/19

(84) Designated Contracting States :
AT BE CH DE FR GB IT LI NL

(56) References cited :
DE-A- 2 030 101
BRIT. MED. J., vol. 1, 1970, pages 568-569,
abstract no. B4956; G. SHERA: "Phage treat-
ment of severe burns"

(73) Proprietor : KAJI, Akira
1-1-9, Daimon-cho Higashikurume-shi
Tokyo 203 (JP)

(72) Inventor : KAJI, Akira
1-1-9, Daimon-cho Higashikurume-shi
Tokyo 203 (JP)

(74) Representative : Eitle, Werner, Dipl.-Ing. et al
Hoffmann, Eitle & Partner Patentanwälte
Arabellastrasse 4
W-8000 München 81 (DE)

Jouve, 18, rue Saint-Denis, 75001 PARIS

## Description

This invention relates to an antiviral agent useful in the treatment of viral diseases such as viral hepatitis.

Viral diseases include a number of diseases such as viral hepatitis, influenza and herpes encephalitis as well as considerably serious ones, for example, adult T cell leukemia and acquired immune deficiency syndrome. Although there have been some remedies developed therefor, for example, nucleic acid analogues and BRM, none of these conventional remedies can exert a complete therapeutic effect thereon. Further, these remedies are accompanied by serious side effects. Therefore, expectant treatments are frequently applied to patients suffering from these diseases.

Thus, an object of the present invention is to provide an antiviral agent which is useful in the treatment of viral diseases and yet accompanied by little side effect.

In order to achieve the above object, several components contained in a phage (bacteriophage), which has neither any infectivity on eucaryotic cells nor pathogenicity, has been isolated and purified, and the antiviral effect of each component has been also examined. As a result, it has been found that a phage single-stranded DNA of a single-stranded DNA phage has a high antiviral activity. Thus, the present invention has been completed based on the above findings.

Accordingly, the present invention provides an antiviral agent comprising a phage single-stranded DNA of a single-stranded DNA phage as an active ingredient.

Typical examples of the phage single-stranded DNA of the single-stranded DNA phase as described above are those as will be shown in Examples hereinafter.

The phage single-stranded DNA of the single-stranded DNA phage according to the present invention exerts an excellent effect as an antiviral agent. Thus, the present invention provides an epoch-making antiviral agent for treating viral diseases for which there has been developed no effective and safe remedy.

The phase single-stranded DNA of the single-stranded DNA phage to be used in the antiviral agent of the present invention may be obtained by, for example, the following process.

A bacterium is transformed by introducing a commercially available phage-replicative form doublestranded DNA or phage single-stranded DNA thereto by, for example, treating with calcium ion. The transformant thus obtained is incubated and then the cells are removed by, for example, centrifuging. The resulting single-stranded DNA phage suspension is concentrated and proteins originating from the phage are removed therefrom. Subsequently, the aimed phage single-stranded DNA of the single-stranded DNA phage is obtained by precipitating with, for example, ethanol.

Although, the single-stranded DNA phage as used herein may originate from any source, it is preferable to employ a filamentous phage which is highly infectious for a host bacterium; shows a high multiplication rate of the single-stranded DNA phage per se; does not break the host cell; is liberated out of the host cell and does not significantly suppress the multiplication of the host cell.

A process for preparing a phage single-stranded DNA of a single-stranded DNA phage in accordance with the present invention will be now described in detail.

Examples of the commercially available phage-replicative form double-stranded DNA include M13mp-7RFDNA, M13mp8RFDNA, M13mp9RFDNA, M13mp10RFDNA and M13mp11RFDNA (each obtained from Pharmacia Co., Ltd.); M13mp18RFDNA, M13mp19DNA and $\phi$X174RFIDNA (each obtained from Takara Co., Ltd.); and M13mp20RFDNA and M13mp21DNA (each obtained from IBI). Examples of the phage single-stranded DNA include M13mp7SSDNA and M13mp18SSDNA (each obtained from Pharmacia Co., Ltd.); M13mp-19SSDNA and $\phi$X174SSDNA (each obtained from Bethesda Research Laboratories); and M13mp9SSDNA (obtained from Boehringer-Mannheim).

When a phage-replicative form double-stranded DNA is to be used, in particular, it may be preliminarily modified by conventional recombinant DNA techniques. Namely, the phage-replicative form double-stranded DNA may be incised with an appropriate restriction enzyme and a preliminarily prepared heterologous gene fragment may be introduced thereto by using ligase to thereby elongate, shorten or rearrange the double-stranded DNA chain.

Transformation through calcium co-precipitation may be effected in a conventional manner by using, for example, calcium chloride. Preferable examples of the host bacterial cell are Escherichia coli and Bacillus subtilis which are highly safe. The former is still preferable. Among bacteria belonging to E. coli, those having F-pilus to be infected with a filamentous Phage are particularly preferable, since the aimed phage single-stranded DNA can be readily separated.

The bacterial cells thus transformed is then incubated in a medium. Although the medium is to be selected depending on the employed bacterium, anyone may be used as long as it contains a carbon source, a nitrogen source and inorganic materials required for the growth of the bacterium. An example thereof is YT medium which contains Bacto trypton, Bacto yeast extract, sodium chloride and distilled water. The incubation is effected at 35 to 39°C for 12 to 36 hours.

After the completion of the incubation, a single-stranded DNA phage suspension may be obtained by, for example, centrifuging the incubation medium.

The single-stranded DNA phage suspension thus obtained is added to a suitable host cell, which has been preferably precultured to the logarithmic growth phase, to thereby infect the latter with the former preferably at a multiplicity of 1 to 10. Then, the infected cell is incubated under the same conditions as those employed above. Thus, a fresh single-stranded DNA phage suspension can be obtained. A single-stranded DNA phage suspension can be efficiently prepared by repeating the above procedure.

Alternately, a single-stranded DNA phage available in public such as $\phi$X174 or fl (each obtained from Institute for Fermentation, Osaka); or M13, fd, S13, $\phi$R or ZJ/2 (each obtained from ATCC) may be added to a suitable host cell, which has been preferably precultured to the logarithmic growth phase, to thereby infect the latter with the former preferably at a multiplicity of 3 to 8. Then, the infected cell is incubated under the same conditions as those described above. Thus, the aimed single-stranded DNA phage suspension can be obtained without using any commercially available phage-replicative form double-stranded DNA or phage single-stranded DNA.

The single-stranded DNA phage suspension thus obtained is treated with polyethylene glycol and salt(s) to thereby precipitate the single-stranded DNA phage which is then concentrated.

In order to remove proteins from the single-stranded DNA phage, the proteins,may be precipitated by using, for example, phenol, chloroform or isoamyl alcohol and then centrifuged.

Finally, the phage single-stranded DNA is precipitated by conventional ethanol-precipitation. It is preferable to further purify the resulting precipitate by, for example, dialysis against sterile water or a 0.1% saline solution, or by gel filtration column chromatography with the use of a suitable gel, e. g:, Sephadex.

The phage single-stranded DNA thus obtained may be lyophilized and stored.

To illustrate the preparation of the phage single-stranded DNA of the single-stranded DNA phage, which is the active ingredient of the antiviral agent according to the present invention, the following Examples will be given.

## EXAMPLE 1

### EXAMPLE 1-1

3 m$\ell$ of a suspension of E. coli K-12 JM101 strain (obtained from Pharmacia Co., Ltd.) was incubated in 200 m$\ell$ of YT medium, which had been prepared by dissolving 8 g of Bacto trypton (obtained from Difco), 5 g of Bacto yeast extract (obtained from Difco) and 5 g of sodium chloride in distilled water to give a total volume of 1 $\ell$ and adjusting the pH value of the resulting solution to 7.2, for approximately one hour until the UV aborbance at 550 nm of the culture suspension reached approximately 0.2. Then, the culture suspension was cooled in an ice-bath at 4°C and centrifuged at 9,000 g for ten minutes at 4°C. After removing the supernatant, a precipitate containing the multiplied E. coli was obtained in the form of pellets. These pellets were dissolved in 90 m$\ell$ of a solvent mixture which comprised a 1 M aqueous solution of sodium chloride, 1 M manganese chloride and a 12.5 mM aqueous solution of sodium acetate (pH 5.6) and distilled water at a ratio of 1:5:80:14. The resulting solution was incubated in an ice-bath at 4°C for 20 minutes and then centrifuged at 9,000 g for ten minutes at 4°C. Thus, impurities including the incubation medium were removed as the supernatant. The residual precipitate was suspended in 9 m$\ell$ of a solvent mixture which comprised a 1 M aqueous solution of calcium chloride, 1 M manganese chloride, a 12.5 mM aqueous solution of sodium acetate (pH 5.6) and distilled water at a ratio of 3:4:32:1. To the suspension thus obtained, 15 $\mu\ell$ of a solution obtained by dissolving M13mg8RF1DNA (obtained from Pharmacia Co., Ltd) in TE buffer comprising 10 mM of Tris hydrochloride (pH 8.0) and 1mM EDTA to give the final concentration of 1 ng/$\mu\ell$ was added. The resulting mixture was incubated in an ice-bath at 4°C for 20 minutes and then in a water-bath at 37°C for two minutes to thereby effect the transfection of the E. coli strain.

### EXAMPLE 1-2:

100 $\mu\ell$ of a reaction mixture containing the E. coli transformant as obtained in Example 1-1 was added to 3 m$\ell$ of a soft agar medium. 50 $\mu\ell$ of a 2% X-gal (obtained from Bethesda Research Laboratories) in dimethylformamide and 10 $\mu\ell$ of a 100 mM aqueous solution of IPTG (obtained from Bethesda Research Laboratories) were further added thereto. The resulting mixture was poured on YT agar medium and incubated overnight at 37°C. Thus, the E. coli infected with the phage formed blue plaques. Among these plaques, one was taken and diluted 100-fold and 10O,000-fold. 10 $\mu\ell$ of each diluted solution was added to 3 m$\ell$ of a soft agar medium and 50 $\mu\ell$ of a 2% X-gal (obtained from Bethesda Research Laboratories) solution in dimethylformamide and

10 μℓ of a 100 mM aqueous solution of IPTG (obtained from Bethesda Research Laboratories) were further added thereto. The resulting mixture was poured on YT agar medium and incubated at 37°C overnight. The surface of the medium, which was thus completely coated with blue plaques, was scratched off together with the plaques and centrifuged at 10,000 g for ten minutes at room temperature. Thus, a phage suspension was obtained as the supernatant. 7,500 μℓ of a suspension of E. coli K-12 JM101 strain (obtained from Pharmacia Co., Ltd.) of a concentration of $10^9$ cells/mℓ was incubated in 15,000 mℓ of YT medium at 37°C for two to three hours until the UV absorbance at 550 nm of the Culture suspension reached 0.4. To the resulting culture suspension ($2 \times 10^8$ cells/mℓ), the above phage suspension was added to thereby infect the former with the latter at a multiplicity of 5 and then the resulting mixture was incubated for seven hours.

EXAMPLE 1-3:

The culture suspension as obtained in Example 12 was centrifuged at 10,000 g for ten minutes at room temperature. To the supernatant thus obtained, PEG 6,000 (obtained from Wako Pure Chemicals Co., Ltd.) and sodium chloride were added in such a manner as to give concentrations of 4 wt% and 0.55 M, respectively. The obtained mixture was allowed to stand overnight at 4°C. Then, it was centrifuged at 12,000 g for 20 minutes at 4°C and the supernatant was discarded. TE buffer (pH 8) was added to the precipitate which was thus suspended therein. To the obtained suspension, the equivalent volume of phenol was added. The resulting mixture was thoroughly stirred and centrifuged to thereby divide the same into two phases. After removing the phenol phase, a solvent mixture comprising phenol and chloroform containing isoamyl alcohol in an amount corresponding to 1/24 in volume thereof (1:1) was added to the aqueous phase. The obtained mixture was thoroughly stirred and centrifuged to divide the same into two phases. After removing the solvent mixture phase, the equivalent volume of chloroform containing isoamyl alcohol in an amount corresponding to 1/24 in volume thereof was added to the aqueous phase. The obtained mixture was throughly stirred and centrifuged to thereby divide the same into two phases. After removing the chloroform phase, diethyl ether was added to the remaining aqueous phase. The resulting mixture was thoroughly stirred and centrifuged to divide the same into two phases. Then, the diethyl ether phase was removed. After repeating this procedure five times, the obtained aqueous solution was incubated at 50°C for 30 minutes to remove the residual diethyl ether. The aqueous solution was subjected to ethanol-precipitation by adding twice as much as cold ethanol and 3 M sodium acetate corresponding to 1/10 in volume of the same thereto. To the precipitate thus formed, 40 mℓ of sterile water was added arid the mixture was dialyzed in a dialysis tube for 46 hours while exchanging the external solution (8 ℓ) seven times. Then, it was lyophilized to give 80 mg of M13mp8 phage single-stranded DNA.

EXAMPLE 2

100 μℓ of a suspension of E. coli K-12 JM101strain (obtained from Pharmacia Co., Ltd.; $10^9$ cells/mℓ) was incubated in 200 mℓ of YT medium at 37°C for two to three hours until the UV absorbance at 550 nm of the culture suspension reached 0.4. To the culture suspension ($2 \times 10^8$ cells/mℓ) thus obtained, the phage solution as prepared in Example 1-2 was added in such a manner as to give a multiplicity of infection of 5. The resulting mixture was incubated for seven hours and then the incubation medium was separated and purified in the same manner as the one described in Example 1-3. Thus, 100 mg of M13mp8 phage single-stranded DNA was obtained.

EXAMPLE 3

EXAMPLE 3-1:

M13mp8RF1DNA (obtained from Pharmacia Co., Ltd.) was incised with a restriction enzyme EcoRI (obtained from Pharmacia Co., Ltd.) and subjected to ethanol-precipitation at -20°C. Then, it was dissolved in 10 μℓ of TE buffer. Separately, Charon 27 phage DNA, wherein a gene fragment of duck hepatitis B virus (DHBV) was inserted as an heterologous gene at the EcoRI recognition site, was incised EcoRI. The aimed DHBV gene fragment was recovered by 0.5% agarose gel electrophoresis and then dissolved in TE buffer. 200 ng of the M13mg8RF1DNA EcoRI fragment and 500 ng of the DHBV gene fragment thus obtained were mixed with 400 U of T4DNA ligase (obtained from New England Bio Labo.) and the resulting mixture was allowed to react in a ligation buffer for four hours at 16°C. Thus, a reaction mixture containing a phage-replicative form double-stranded DNA, wherein the DHBV gene fragment was inserted, was obtained.

EXAMPLE 3-2:

E. coli was transformed with the reaction mixture as obtained in Example 3-1 in the same manner as the one described in Example 1-1. The reaction mixture containing the transformant thus obtained was added to a soft agar medium. Further, 50 $\mu\ell$ of a 2% X-gal (obtained from Bethesda Research Laboratories) solution in dimethylformamide and 10 $\mu\ell$ of a 100 mM aqueous solution of IPTG (obtained from Bethesda Research Laboratories) were added thereto. The resulting mixture was placed on YT agar medium and incubated at 37°C overnight. Thus, white plaques comprising E. coli transfected with the recombinant phage were formed. Several plaques thus formed were taken and each one was added to 10 m$\ell$ of a suspension of E. coli K-12 JM101 strain (obtained from Pharmacia Co., Ltd.) of a concentration of $10^9$ cells/m$\ell$ diluted 200-fold which had been preliminarily incubated in YT medium overnight. After incubating the mixture overnight, the culture suspension was centrifuged at 12,000 g for ten minutes at room temperature to thereby divide the same into the supernatant and precipitate. The phage-replicative form double-stranded DNA thus multiplied was purified by the conventional method from the E. coli transformant contained in the precipitate and incised with EcoRI. The fragment thus obtained was examined by agarose gel electrophoresis and thus found to agree with the DHBV gene fragment as described in Example 3-1.

EXAMPLE 3-3:

The supernatant as obtained in Example 3-2 was diluted 100-fold and 10,000-fold and added to 3 m$\ell$ portions of a soft agar medium. 50 $\mu\ell$ of a 2% X-gal (obtained from Bethesda Research Laboratories) solution in dimethylformamide and 10 $\mu\ell$ of a 100mM aqueous solution of IPTG (obtained from Bethesda Research Laboratories) were further added to each mixture. The resulting mixture was placed on YT agar medium and incubated at 37°C overnight. Then, the soft agar medium, whose surface was completely coated with white plaques, was scratched together with the plaques and centrifuged at 12,000 g for ten minutes at room temperature. Thus, a phage suspension was obtained as the supernatant. 7,500 $\mu\ell$ of a suspension of E. coli K-12 JM101 strain (obtained from Pharmacia Co., Ltd.) of a concentration of $10^9$ cells/m$\ell$ was incubated in 15,000 m$\ell$ of YT medium at 37°C for two to three hours until the UV absorbance at 550 nm of the culture suspension reached 0.4. To the culture suspension ($2\times10^8$ cells/m$\ell$), the above phage suspension was added in such a manner as to give a multiplicity of 5. The mixture was incubated for seven hours and then the phage suspension was separated and purified in the same manner as the one described in Example 1-3. Thus, 100 mg of M13mp8 phage single-stranded DNA, wherein the DHBV gene fraction was inserted into the restriction enzyme EcoRI recognition site as an heterologous gene, was obtained.

EXAMPLE 4

E. coli K-12 JM101 strain (obtained from Pharmacia Co., Ltd.) was cultured in 15 $\ell$ of YT medium until the UV absorbance at 550 nm of the culture suspension reached 0.4. To the culture suspension ($2\times10^8$ cells/m$\ell$), the phage suspension as obtained in Example 3 was added in such a manner as to give a multiplicity of 5. The mixture was incubated seven hours arid then the phase suspension was separated and purified in the same manner as the one described in Example 1-3. Thus, 120 mg of M13mp8 phage single-stranded DNA, wherein the DBBV gene fraction was inserted into the restriction enzyme EcoRI recognition site as an heterologous gene, was obtained.

EXAMPLE 5

E. coli K-12 JM101 strain (obtained from Pharmacia Co., Ltd.) was cultured in 6 $\ell$ of YT medium until UV absorbance at 550 nm of the culture suspension reacted 0.2. To the culture suspension ($2\times10^8$ cells/m$\ell$), a suspension of M13 phage wild strain (ATCC #15669-B1) was added in such a manner as to give a multiplicity of 10. The resulting mixture was incubated for eight hours and then the phage suspension was separated and purified in the same manner as the one described in Example 1-3. Thus, 80 mg of phage single-stranded DNA of the M13 phage wild strain was obtained.

In order to illustrate the effect of the antiviral agent of the present invention in treating viral diseases, the following Experimental Examples will be given.

EXPERIMENTAL EXAMPLE 1

Inhibitory effect on the multiplication of DHBV:

Duck serum infected with DHBV was diluted five-fold with a physiological saline solution. The obtained solution was intravenously injected into ducks aged 0 day at a dose of 50 $\mu\ell$. These ducks were divided into five groups each having five birds. The phage single-stranded DNAs as obtained in the abovementioned Examples 2, 4 and 5 were administered to separate groups. Further, a single-stranded DNA obtained by denaturing commercially available calf thymus DNA was administered to another one group. Each single-stranded DNA was dissolved in a physiological saline solution at the use and intravenously administered to the ducks once a day for nine days starting from the day of the administration of the duck serum infected with DHBV. From the fourth days, the blood of each bird was collected from the heart on every other day. The residual one group was employed as a control group to which the physiological saline solution alone was intravenously administered.

Table 1 shows the effect of each single-stranded DNA of inhibiting the increase in virus DNA in blood from the fourth to tenth days following the administration of the duck serum infected with DHBV.

## Table 1

### Inhibitory ratio on increase in virus DNA in blood (%)

| Sample | Inhibitory Ratio on Increase in Virus DNA in Blood (%) |
|---|---|
| Phage single-stranded DNA obtained in Example 2 | 100 |
| Phage single-stranded DNA obtained in Example 4 | 100 |
| Phage single-stranded DNA obtained in Example 5 | 100 |
| Single-stranded DNA obtained by denaturing calf thymus DNA | 0 |
| Control | 0 |

Note: Each sample was administered at a dose of 2.5 mg/kg.

These results suggest that each antiviral agent of the present invention is effective in treating a viral disease.

In addition, the administration of the products of the Examples 2, 4 and 5, which are active ingredients of the antiviral agent of the present invention, would exert neither any toxicity nor side effect on the ducks at the effective dose as defined above.

When the product of Example 2 was intravenously administered to male Balb/c mice at a dose of 37 mg/kg, no animal died. Thus, it has been proved that it is also safe at this dose.

From these results, it is estimated that the effective dose of the antiviral agent of the present invention might range from 0.01 to 50 mg, preferably 0.1 to 10 mg, per kg body weight of an adult.

Thus, the effect of the present invention has been described by the above Examples. However, it is expected that any single-stranded DNA has similar pharmacological properties, as long as it satisfies the abovementioned requirements.

The single-stranded DNA, which is the active ingredient of the antiviral agent of the present invention, may be used in formulating various preparations including injection, solution, ointment and suppository by simultaneously employing appropriate materials selected from among, for example, solvent, excipient and auxiliary in a conventional manner.

At the formulation of these preparations, it may be blended with other pharmaceutically active ingredients.

An injection or a solution may be prepared by using conventional diluents such as distilled water for injection, physiological saline solution, aqueous solution of dextrose, vegetable oil for injection, propylene glycol or polyethylene glycol. Other additives such as tonicity agent, stabilizer, preservative or soothing agent may be further used, if required. It is preferable in this case to dissolved the active ingredient in a sterile solvent for injection.

An ointment may be prepared by using, for example, fat, oil, lanolin, petrolatum, paraffin, wax, resin, plastics, glycol, higher alcohol, glycerol, emulsifier or suspending agent. Other additives such as stabilizer or preservative may be further used, if required. It is preferable in this case to use a sterile medium for ointment.

A suppository may be prepared by using, for example, cacao fat or macrogol. Other additives such as stabilizer or preservative may be further used, if required. It is preferable in this case to use a sterile medium for suppository.

To further illustrate the present invention, and not by way of limitation, the following Application Examples will be given.

## APPLICATION EXAMLE 1

100 mg of the M13mp8 phage single-stranded DNA as obtained in Example 2 was filled in a sterile vial and sealed. At the use, it was dissolved in a 5% glucose solution or a physiological saline solution and intravenously administered dropwise to a patient for 0.5 to 2 hours per 500 m$\ell$ of transfusion.

## APPLICATION EXAMPLE 2

100 mg of the M13mp8 phage single-stranded DNA, wherein a DHBV DNA fragment was inserted, as obtained in Example 4 was filled in a sterile vial and sealed. At the use, it was dissolved in a 5% glucose solution or a physiological saline solution and intravenously administered dropwise to a patient for 0.5 to 2 hours per 500 m$\ell$ of transfusion.

## APPLICATION EXAMPLE 3

100 mg of the single-stranded DNA of the M13 phage wild strain as obtained in Example 5 was filled in a sterile vial and sealed. At the use, it was dissolved in a 5% glucose solution or a physiological saline solution and intravenously administered dropwise to a patient for 0.5 to 2 hours per 500 m$\ell$ of transfusion.

**Claims**

1. An antiviral agent comprising a plage single-stranded DNA of a single-stranded DNA plage as an active ingredient.

**Patentansprüche**

1. Antivirusmittel, umfassend eine Phagen-Einzelstrang-DNA eines Einzelstrang-DNA-Phagen als aktiven Bestandteil.

**Revendications**

1. Agent antiviral comprenant un ADN monocaténaire phagique d'un phage à ADN monocaténaire comme ingrédient actif.